# EUROPÄISCHE PATENTSCHRIFT

(11) **EP 1 792 603 B1**
(45) Veröffentlichungstag und Bekanntmachung des Hinweises auf die Patenterteilung: **26.01.2011**
(21) Anmeldenummer: 06121502.6
(22) Anmeldetag: 29.09.2006
(51) Int. Cl.: A61K 8/34, A61K 8/04, A61Q 19/00

(54) **Kosmetische O/W-Emulsion mit 1,2-Hexandiol**
Cosmetic O/W-emulsion comprising 1,2-hexanediol.
Une émulsion cosmétique huile/eau comprenant 1,2-hexanediol

(30) Priorität: 25.10.2005 DE 102005051864
(43) Veröffentlichungstag der Anmeldung: 06.06.2007
(73) Patentinhaber: Beiersdorf AG, 20253 Hamburg (DE)
(72) Erfinder: Behrens, Svea, 21109, Hamburg (DE); Bleckmann, Andreas, 22926, Ahrensburg (DE); Kröpke, Rainer, 22869, Schenefeld (DE); Meiring, Uta, 21077, Hamburg (DE); Nielsen, Jens, 25448, Henstedt-Ulzburg (DE)

(56) Entgegenhaltungen:
- EP-A- 1 426 029
- WO-A-03/069994
- WO-A-2004/050045
- WO-A-2006/045743
- WO-A2-2006/082151

## Beschreibung

Die vorliegende Erfindung betrifft eine kosmetische Zubereitung enthaltend 1,2-Hexandiol wie in Anspruch 1 definiert.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z. B. das Entstehen von Fältchen, Falten) verhindern und vermindern sollen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um Emulsionen über einen längeren Zeitraum stabil zu halten und eine Entmischung der Phasen zu verhindern, werden den Emulsionen sogenannte Emulgatoren zugesetzt. Bei Emulgatoren handelt es sich in der Regel um Moleküle mit einem polaren, hydrophilen Strukturelement und einem unpolaren, lipophilen Strukturelement. Ende der vierziger Jahre wurde ein System entwickelt, das die Auswahl von Emulgatoren erleichtern sollte. Jedem Emulgator wird ein sogenannter HLB-Wert (eine dimensionslose Zahl zwischen 0 und 20) zugeschrieben, der angibt, ob eine bevorzugte Wasser- oder Öllöslichkeit vorliegt. Zahlen unter 9 kennzeichnen öllösliche, hydrophobe Emulgatoren, Zahlen über 10 wasserlösliche, hydrophile.

Bei der Herstellung einer Emulsion wird die Wasserphase mit der Lipidphase (Ölphase) unter Rühren vereinigt, wobei die Tröpfchen der inneren Phase der Emulsion auf eine Größe von unter 10 µm zerkleinert werden müssen, damit die Emulsion stabil wird.

Bei der Herstellung von O/W-Emulsionen mit hydrophilen Emulgatoren (Emulgatoren mit einem HLB-Wert von größer/gleich 10) tritt häufig das Problem auf, dass bei der Verwendung von überwiegend mittelpolaren bis polaren Lipiden in der Ölphase (d.h. Lipide mit einer Grenzflächenspannung gegen Wasser von kleiner als 30 mN/m) eine unerwünschte Phasenumkehr stattfinden kann. Ferner neigen derartige O/W-Emulsionen bei der Lagerung zur Phasenumkehr, d. h. die Zubereitungen sind häufig nicht besonders lagerstabil.

Es war daher die Aufgabe der vorliegenden Erfindung, eine O/W-Emulsion mit hydrophilen Emulgatoren zu entwickeln, deren Neigung zur Phasenumkehr während der Herstellung und/oder Lagerung deutlich reduziert ist.

Überraschend gelöst wird die Aufgabe durch eine kosmetische Öl-in-Wasser-Emulsion (O/W-Emulsion) enthaltend 1,2-Hexandiol sowie, als O/W-Emulgatoren Glycerylstearatcitrat und/oder Polyglycerylmethylglucosedistearat mit Ausnahme der folgenden Rezepturen:

| INCl | Produktname | Hersteller | Gew.-% | Gew.-% |
|---|---|---|---|---|
| Glyceryl stearate citrate | Dracorin CE | Symrise | 4,0 | 4,0 |
| Srearyl Heptanoate, Stearyl caprylate | PCL solid | Symrise | 3,0 | 3,0 |
| Paraffinum Liquidum | Paraffin oil °E | Parafluid | 7,0 | 7,0 |
| Stearyl Alcohol | Lanette 18 | Cognis | 1,5 | 1,5 |
| Glyceryl Stearate | Dracorin GMS | Symrise | 1,5 | 1,5 |
| Dimethicone | Dow Corning 200 fluid | DowComing | 2,0 | 2,0 |
| Water | | | Ad. 100 | Ad. 100 |
| Carbomer | Carbopol ETD 2050 Polymer | Noveon | 0,15 | 0,15 |
| 1,2-Hexandiol | 1,2-Hexandiol | Symrise | 0,25 | 0,15 |
| Caprylyl Glycol | Caprylyl Glycol | Symrise | 0,25 | 0,15 |
| 2-Hydroxy-2,4,6-cycloheptatrienon | Tropolone | Symrise | | 0,01 |
| Amino Methylpropanol | Neutralizer AMP-95 | Dow/Agnus | 0,1 | 0,1 |

| | | | | |
|---|---|---|---|---|
| pH-Wert: 5,5. | | | | |

Erfindungsgemäß vorteilhaft wird die Aufgabe femer gelöst durch ein Verfahren zur Herstellung einer kosmetischen Öl-in-Wasser-Emulsion, dadurch gekennzeichnet, dass 1,2-Hexandiol a) vor dem Aufheizen in die Wasserphase zugegeben wird oder b) beim Abkühlvorgang in die sich ausbildende Emulsion bei ca. 30°C bis 40°C - vorzugsweise bei 35°C zugegeben wird.

Erfindungsgemäß vorteilhaft ist dabei auch eine Emulsion, die nach dem erfindungsgemäß vorteilhaften Verfahren hergestellt ist.

Erfindungsgemäß vorteilhaft ist die Verwendung von 1,2-Hexandiol als Emulgierhilfe für kosmetische Öl-in-Wasser-Emulsionen.

Der Einsatz von 1,2-Hexandiol in kosmetischen Emulsionen ist dem Fachmann an sich bekannt, doch konnte der Stand der Technik nicht den Weg zur vorliegenden Erfindung weisen.

Die WO 95/01151 offenbart die Verwendung von Alkandiolen mit 5-7 Kohlenstoffatomen in kosmetischen Zubereitungen. O/W-Emulsionen mit 1,2-Hexandiol sind jedoch nicht explizit offenbart. Ebenso sind keine Ausführungsbeispiele mit 1,2-Hexandiol und/oder mit den erfindungsgemäßen Emulgatoren offenbart.

Die DE 103 41 179 offenbart Deodorantzusammensetzungen mit einer Kombination aus Alkan-1,2-diolen und α- und/oder β-Hydroxysäuren. O/W-Emulsionen mit 1,2-Hexandiol und den erfindungsgemäßen Emulgatoren sind jedoch nicht offenbart.

Die DE 102 05 190 offenbart kosmetische Zubereitungen mit einer Kombination aus Polyolen und einem oder mehreren Diolen, u.a. Hexandiol. Diese Schrift offenbart jedoch kein 1,2-Hexandiol in einer O/W-Emulsion.

Die EP 1 078 638 offenbart Lichtschutzzubereitungen mit hohen Konzentrationen an 1,2-Hexandiol.

Die EP 1 426 029 offenbart Emulsionen mit 1,2-Alkandiol, einer oberflächenaktiven Verbindung, einer öligen Substanz und Wasser.

Die WO 03/069994 offenbart die Verwendung einer Mischung aus zwei, drei oder mehr unverzweigten Alkandiolen, deren Kettenlängen unterschiedlich sind und jeweils im Bereich von 5 bis 10 C-Atomen liegen, als antimikrobieller Wirkstoff.

Die WO 2004/050045 offenbart eine Zubereitung mit einem oberflächenaktiven Silikonderivat, einem "Co-Surfactant" ausgewählt aus der Gruppe der Verbindungen der Monohydroxyalkohole, organischen Diole, organischen Triole, organischen Tetraole, Silikondiole, Silikontriole, Silikontetraole, kationischen organischen "Surfactants", anionischen organischen "Surfactants", nichtionischen organischen "Surfactants", polaren Ölen und Wasser.

Es ist erfindungsgemäß vorteilhaft, wenn die erfindungsgemäße Emulsion, dadurch gekennzeichnet ist, dass der Gehalt an 1,2-Hexandiol in der Emulsion von 0,3 bis 3,0 Gewichts-% und bevorzugt zwischen 0.5 und 1,5 Gewichts-%. jeweils bezogen auf das Gesamtgewicht der Emulsion, beträgt.

Es ist erfindungsgemäß vorteilhaft, wenn die erfindungsgemäße Emulsion, dadurch gekennzeichnet ist, dass die Emulsion einen oder mehrere Öl-in-Wasser-Emulgatoren (O/W-Emulgatoren) in einer Gesamtkonzentration von 1,5 bis 6,0 Gewichts-% und bevorzugt in einer Gesamtkonzentration von 2,0 bis 4,0 Gewichts-%, jeweils bezogen auf das Gesamtgewicht der Emulsion, beträgt.

Es ist erfindungsgemäß vorteilhaft, wenn die erfindungsgemäßeEmulsion, dadurch gekennzeichnet ist, dass als O/W-Emulgatoren hydrophile Emulgatoren mit einem HLB-Wert von größer/gleich 10 eingesetzt werden. Der HLB-Wert von Emulgatoren kann dabei den üblichen Standartwerken (z. B. Fiedler, Lexikon der Hilfsstoffe für Pharmazie, Kosmetik und angrenzende Gebiete, Editio Cantor Verlag, Aulendorf) entnommen werden.

Es ist erfindungsgemäß bevorzugt, wenn die erfindungsgemäße Emulsion, dadurch gekennzeichnet ist, dass als O/W-Emulgatoren Glycerylstearatcitrat (CAS 39175-72-9, INCI Glyceryl Stearate Citrate, z.B. Imwitor 370 der Firma Hüls), Polyglycerylmethylglucosedistearat (INCI Polyglyceryl Methyl Glucose Distearate, z.B. Tego Care 450 der Firma Goldschmidt) und/oder Polyethylenglycol(2000)monostearat (INCI PEG-40 stearate) eingesetzt werden.

Erfindungsgemäß bevorzugt ist die erfindungsgemäße Emulsion frei von Natriumdihydroxycetylphosphat (Sodium Dihydroxycetyl Phosphate, z. B. Dragophos S).

Es ist erfindungsgemäß vorteilhaft, wenn die erfindungsgemäße Emulsion dadurch charakterisiert sind, dass die Emulsion Tocopherylacetat enthält. Tocopherylacetat ist erfindungsgemäß vorteilhaft in einer Konzentration von 0,1 bis 1,0 Gewichts-% und bevorzugt in einer Konzentration von 0,3 bis 0,7 Gewichts-%, bezogen auf das Gesamtgewicht der Zubereitung, in der Emulsion enthalten.

Es ist erfindungsgemäß vorteilhaft, wenn die erfindungsgemäße Emulsion dadurch charakterisiert sind, dass sie eine oder mehrere Verbindungen gewählt aus der Gruppe der Verbindungen Phenoxyethanol, Methylparaben, Propylparaben enthält. Derartige Verbindungen sind erfindungsgemäß vorteilhaft in einer Einzelkonzentration von 0,1 bis 1,0 Gewichts-% und bevorzugt in einer Einzelkonzentration von 0,3 bis 0,7 Gewichts-%, bezogen auf das Gesamtgewicht der Zubereitung, in der Emulsion enthalten.

Die erfindungsgemäße Emulsion kann die Konsistenz einer Salbe, Creme oder dünnflüssigen Lotion aufweisen bzw. eine Salbe, Creme oder dünnflüssige Lotion darstellen.

Es ist erfindungsgemäß bevorzugt, wenn das erfindungsgemäß vorteilhafte Verfahren in Form des Hot-Hot-Verfahrens durchgeführt wird. Bei diesem Verfahren werden eine heiße Öl- und Wasserphase miteinander vereinigt.

Dabei ist es erfindungsgemäß besonders bevorzugt, wenn das Verfahren bei einer Temperatur nicht über 85 °C durchgeführt wird.

Die Emulsion kann erfindungsgemäß vorteilhaft als Salbe, Creme oder Lotion (ggf. sprühbar) vorliegen.

Die Emulsion kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Emulsion, erfindungsgemäß.

Die Wasserphase der erfindungsgemäßen Emulsion kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner, Repellentien sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der so genannten Carbopole, beispielsweise Carbopole der Typen 980, 981, 1382, 2984, 5984, jeweils einzeln oder in Kombination.

Die erfindungsgemäße Ölphase kann alle herkömmlichen Bestandteile von in der Kosmetik eingesetzten Öl-, Fett- und Wachskomponenten enthalten.

Die erfindungsgemäße Emulsion kann erfindungsgemäß vorteilhaft kosmetische Wirk- und Pflegestoffe enthalten, beispielsweise die nach der Kosmetikverordnung zugelassenen UV-Lichtschutzfilter. Derartige Wirkstoffe können erfindungsgemäß vorteilhaft in Konzentrationen von 0,01 bis 30 Gew.-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gew.-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gew.-%, bezogen auf das Gesamtgewicht der Zubereitung eingesetzt.

Die erfindungsgemäße Zubereitung kann Parfümstoffe in beliebiger Zusammensetzung und Menge enthalten.

Die nachfolgenden Beispiele sollen die vorliegende Erfindung verdeutlichen ohne sie einzuschränken. Die Angaben beziehen sich stets auf Gew.-%, sofern nicht andere Angaben gemacht werden.

**Beispiele**

| Beispiel | 1 | 2 | 3 | 4 | 5 | 6 |
|---|---|---|---|---|---|---|
| Glyceryl Stearate Citrat | 1,0 | | 2,0 | | | 2,0 |
| PEG-40 Stearat | | | | | | |
| Polyglycerylmethylglucose-distearat | | 2,0 | | 3,0 | 1,5 | 0,5 |
| Glycerylstearate | | | | | | |
| Cetyl Alkohol | 0,5 | | 2,0 | 1,5 | 1,0 | |
| Stearyl Alcohol | 0,5 | 0,5 | | | | |
| Cetearyl Alkohol | | | | | | 1,5 |
| Caprylic/Capric Triglycerid | 4,0 | | 5,0 | | 3,0 | 0,5 |
| Ethylhexylcocoat | | 2,0 | | | 1,0 | |
| Octyldodecanol | 1,0 | 3,0 | | 5,0 | 2,0 | |
| Mineral Oil | | 2,0 | | | | |
| Hydriertes Polyisobuten | | | | | | |
| Polydecen | | | 2,0 | | | |
| Cyclomethicon | | | | | | |
| Dimethicon | | | | | | 1,5 |
| Phenyltrimethicon | | | | | | |
| Dicaprylyl Carbonat | 2,0 | | | 2,0 | | 3,5 |
| Natürliche Öle (wie z.B. Jojobaöl / Sonnenblumenöl) | | 3,0 | 0,5 | 1,0 | 2,0 | 2,5 |
| **1,2 Hexandiol** | **0,75** | **2,0** | **0,3** | **1,0** | **0,5** | **0,75** |
| Trisodium EDTA | 0,1 | | 0,05 | | 0,3 | |
| Iminodisuccinat | | 0,1 | | 0,3 | | 0,5 |
| Phenoxyethanol | 0,1 | | 0,5 | 0,7 | | 0,4 |
| Parabene | | 0,3 | | 0,3 | 0,2 | |
| Hexamidin Diisethionat | 0,1 | | | 0,05 | 0,1 | |
| Imidodiazolydynl Urea | | | | | | 0,2 |
| DMDM Hydantoin | | 0,2 | | | | |
| lodopropynyl Butylcarbamat | | | 0,2 | | 0,05 | |
| Glycerin | 3,0 | 7,0 | 8,0 | 15,0 | 0,5 | 2,0 |
| Tocopherylacetat | 0,5 | 0,75 | | | | 1,0 |
| Alcohol Denat. | | 2,5 | | | | 7,5 |
| Wasser | ad 100 | ad 100 | ad 100 | ad 100 | ad 100 | ad 100 |

## Patentansprüche

1. Kosmetische Öl-in-Wasser-Emulsion enthaltend 1,2-Hexandiol sowie, als O/W-Emulgatoren Glycerylstearatcitrat und/oder Polyglycerylmethylglucosedistearat mit Ausnahme der folgenden Rezepturen:
| INCI | Produktname | Hersteller | Gew.-% | Gew.-% |
|---|---|---|---|---|
| Glyceryl stearate citrate | Dracorin CE | Symrise | 4,0 | 4,0 |
| Srearyl Heptanoate, | PCL solid | Symrise | 3,0 | 3,0 |
| Stearyl caprylate | | | | |
| Paraffinum Liquidum | Paraffin oil °E | Parafluid | 7,0 | 7,0 |
| Stearyl Alcohol | Lanette 18 | Cognis | 1,5 | 1,5 |
| Glyceryl Stearate | Dracorin GMS | Symrise | 1.5 | 1,5 |
| Dimethicone | Dow Corning 200 fluid | DowComing | 2,0 | 2,0 |
| Water | | | Ad. 100 | Ad. 100 |
| Carbomer | Carbopol ETD 2050 Polymer | Noveon | 0,15 | 0,15 |
| 1,2-Hexandiol | 1,2-Hexandiol | Symrise | 0,25 | 0,15 |
| Caprylyl Glycol | Caprylyl Glycol | Symrise | 0,25 | 0,15 |
| 2-Hydroxy-2,4,6- | Tropolone | Symrise | | 0,01 |
| cycloheptatrienon | | | | |
| Amino Methylpropanol | Neutralizer AMP-95 | Dow/Agnus | 0,1 | 0,1 |
pH-Wert: 5,5.

2. Emulsion nach Anspruch 1, **dadurch gekennzeichnet, dass** der Gehalt an 1,2-Hexandiol in der Emulsion von 0,3 bis 3,0 Gewichts-%, bezogen auf das Gesamtgewicht der Emulsion, beträgt.

3. Emulsion nach einem der vorhergehenden Ansprüche, **dadurch gekennzeichnet, dass** die Emulsion einen oder mehrere Öl-in-Wasser-Emulgatoren (O/W-Emulgatoren) in einer Gesamtkonzentration von 1,5 bis 6,0 Gew.-%, bezogen auf das Gesamtgewicht der Emulsion, enthält.

4. Emulsion nach einem der vorhergehenden Ansprüche, **dadurch gekennzeichnet, dass** die Emulsion ein oder mehrere Verbindungen gewählt aus der Gruppe der Verbindungen Phenoxyethanol, Methylparaben, Propylparaben enthält.

5. Emulsion nach einem der vorhergehenden Ansprüche, **dadurch gekennzeichnet, dass** die Emulsion Tocopherylacetat enthält.

## Claims

1. Cosmetic oil-in-water emulsion comprising 1,2-hexanediol and, as O/W emulsifiers, glyceryl stearate citrate and/or polyglyceryl methylglucose distearate with the exception of the following formulations:
| INCI | Product name | Manufacturer | % by wt. | % by wt. |
|---|---|---|---|---|
| Glyceryl stearate citrate | Dracorin CE | Symrise | 4.0 | 4.0 |
| Stearyl heptanoate, stearyl caprylate | PCL solid | Symrise | 3.0 | 3.0 |
| Paraffinum liquidum | Paraffin oil °E | Parafluid | 7.0 | 7.0 |
| Stearyl alcohol | Lanette 18 | Cognis | 1.5 | 1.5 |
| Glyceryl stearate | Dracorin GMS | Symrise | 1.5 | 1.5 |
| Dimethicone | Dow Corning 200 fluid | DowCorning | 2.0 | 2.0 |
| Water | | | ad 100 | ad 100 |
| Carbomer | Carbopol ETD 2050 polymer | Noveon | 0.15 | 0.15 |
| 1,2-Hexanediol | 1,2-Hexandediol | Symrise | 0.25 | 0.15 |
| Caprylyl glycol | Caprylyl glycol | Symrise | 0.25 | 0.15 |
| 2-Hydroxy-2,4,6-cycloheptatrienone | Tropolone | Symrise | | 0.01 |
| Amino methylpropanol | Neutralizer AMP-95 | Dow/Agnus | 0.1 | 0.1 |
pH: 5.5.

2. Emulsion according to Claim 1, **characterized in that** the content of 1,2-hexanediol in the emulsion is from 0.3 to 3.0% by weight, based on the total weight of the emulsion.

3. Emulsion according to one of the preceding claims, **characterized in that** the emulsion comprises one or more oil-in-water emulsifiers (O/W emulsifiers) in a total concentration of from 1.5 to 6.0% by weight, based on the total weight of the emulsion.

4. Emulsion according to one of the preceding claims, **characterized in that** the emulsion comprises one or more compounds selected from the group of the compounds phenoxyethanol, methylparaben, propylparaben.

5. Emulsion according to one of the preceding claims, **characterized in that** the emulsion comprises tocopheryl acetate.

## Revendications

1. Emulsion cosmétique huile-dans-eau contenant du 1,2-hexanediol ainsi que, comme émulsifiants H/E, du stéarate-citrate de glycéryle et/ou du polyglycéryl-méthylglucose-distéarate, à l'exception des suivants :
| INCl | Nom du produit | Fabricant | % en poids | % en poids |
|---|---|---|---|---|
| Glyceryl stearate citrate | Dracorin CE | Symrise | 4,0 | 4,0 |
| Stearyl Heptanoate, Stearyl caprylate | PCL solid | Symrise | 3,0 | 3,0 |
| Paraffinum Liquidum | Paraffin oil °E | Parafluid | 7,0 | 7,0 |
| Stearyl | Alcohol Lanette 18 | Cognis | 1,5 | 1,5 |
| Glyceryl Stearate | Dracorin GMS | Symrise | 1,5 | 1,5 |
| Dimethicone | Dow Corning 200 fluid | Dow Corning | 2,0 | 2,0 |
| Water | | | Ad. 100 | Ad. 100 |
| Carbomer | Carbopol ETD 2050 Polymer | Noveon | 0,15 | 0,15 |
| 1,2-Hexandiol | 1,2-Hexandiol | Symrise | 0,25 | 0,15 |
| Caprylyl Glycol | Caprylyl Glycol | Symrise | 0,25 | 0,15 |
| 2-Hydroxy-2,4,6-cycloheptatrienone | Tropolone | Symrise | | 0,01 |
| Amino Methylpropanol | Neutralizer AMP-95 | Dow/Agnus | 0,1 | 0,1 |
pH 5,5.

2. Emulsion selon la revendication 1, **caractérisée en ce que** la teneur en 1,2-hexanediol dans l'émulsion est de 0,3 à 3,0% en poids par rapport au poids total de l'émulsion.

3. Emulsion selon l'une quelconque des revendications précédentes, **caractérisée en ce que** l'émulsion contient un ou plusieurs émulsifiants huile-dans-eau (émulsifiants H/E) en une concentration totale de 1,5 à 6,0% en poids par rapport au poids total de l'émulsion.

4. Emulsion selon l'une quelconque des revendications précédentes, **caractérisée en ce que** l'émulsion contient un ou plusieurs composés choisis dans le groupe des composés phénoxyéthanol, méthylparabène, propylparabéne.

5. Emulsion selon l'une quelconque des revendications précédentes, **caractérisée en ce que** l'émulsion contient de l'acétate de tocophéryle.
